# EUROPEAN PATENT APPLICATION

(11) **EP 4 843 502 A2**
(43) Date of publication of application: **25.11.2026**
(21) Application number: 26175023.6
(22) Date of filing: 28.04.2026
(51) Int. Cl.: A61B 5/344, A61B 5/35, A61B 5/256, A61B 5/282, A61B 5/304, A61B 5/327

(54) **ADAPTIVE ELECTRODE SELECTION FOR ABDOMINAL MATERNAL ELECTROCARDIOGRAM**

(30) Priority: 22.05.2025 US 202519215588
(71) Applicant: GE Precision Healthcare LLC, Waukesha, WI 53188 (US)
(72) Inventor: JONNADULA, Bharathkumar, Waukesha, 53188 (US); BOGINENI, Kiran, Waukesha, 53188 (US); M S, Sivakumar, Waukesha, 53188 (US); SETHUMADHAVAN, Nagapriya Kavoori, Waukesha, 53188 (US)
(74) Representative: Kilburn & Strode LLP

(57) **Abstract**

A system for adaptive electrode selection for abdominal maternal electrocardiogram, including a processor and a memory. The memory includes instructions executable by the processor to determine impedance values for electrodes of an abdominal electrode device in contact with an abdomen, determine a lowest impedance value, a second lowest impedance value, and a third lowest impedance value by comparing the plurality of impedance values, and generate an abdominal maternal electrodcardiogram by using a lowest impedance electrode having the lowest impedance value, a second lowest impedance electrode having the second lowest impedance value, and a third lowest impedance electrode having the third lowest impedance value..

## Description

### BACKGROUND

The present disclosure generally relates to adaptive electrode selection, and more specifically to adaptive electrode selection for abdominal maternal electrocardiogram.

An abdominal maternal electrocardiogram (ECG) is a test typically used for a pregnant patient, and may measure the electrical activity of the maternal heart using a probe that is placed on the maternal abdomen. The probe may include a number of electrodes that interface with the skin of the patient and acquire electric biopotentials from the maternal heart.

### SUMMARY

This Summary is provided to introduce a selection of concepts that are further described below in the Detailed Description. This Summary is not intended to identify key or essential features of the claimed subject matter, nor is it intended to be used as an aid in limiting the scope of the claimed subject matter.

A system for adaptive electrode selection for abdominal maternal electrocardiogram, including a processor and a memory. The memory includes instructions executable by the processor to determine impedance values for electrodes of an abdominal electrode device in contact with an abdomen, determine a lowest impedance value of the impedance values, a second lowest impedance value of the impedance values, and a third lowest impedance value of the impedance values by comparing the impedance values, generate an abdominal maternal electrodcardiogram by using a lowest impedance electrode having the lowest impedance value, a second lowest impedance electrode having the second lowest impedance value, and a third lowest impedance electrode having the third lowest impedance value.

In some embodiments, the instructions are executable by the processor to determine that a noise level at a predetermined frequency band exceeds a predetermined noise threshold, and in response to the determination, determine a second plurality of impedance values for the corresponding plurality of electrodes for the abdominal electrode device in contact with the abdomen, determine the lowest impedance value of the second plurality of impedance values, the second lowest impedance value of the second plurality of impedance values, and the third lowest impedance value of the second plurality of impedance values by comparing the second plurality of impedance values, and generate the abdominal maternal electrodcardiogram by using the lowest impedance electrode, the second lowest impedance electrode, and the third lowest impedance electrode.

In some embodiments, the instructions are executable by the processor to determine that a noise level at a predetermined frequency occurs over a period of time exceeding a predetermined time threshold, in response to the determination, determine a second plurality of impedance values for the corresponding plurality of electrodes for the abdominal electrode device in contact with the abdomen, determine the lowest impedance value of the second plurality of impedance values, the second lowest impedance value of the second plurality of impedance values, and the third lowest impedance value of the second plurality of impedance values by comparing the second plurality of impedance values, and generate the abdominal maternal electrodcardiogram by using the lowest impedance electrode, the second lowest impedance electrode, and the third lowest impedance electrode.

In some embodiments, the instructions are executable by the processor to determine that a signal loss occurs beyond a predetermined time threshold, in response to the determination, determine a second plurality of impedance values for the corresponding plurality of electrodes for the abdominal electrode device in contact with the abdomen, determine the lowest impedance value of the second plurality of impedance values, the second lowest impedance value of the second plurality of impedance values, and the third lowest impedance value of the second plurality of impedance values by comparing the second plurality of impedance values, and generate the abdominal maternal electrodcardiogram by using the lowest impedance electrode, the second lowest impedance electrode, and the third lowest impedance electrode.

In some embodiments, the lowest impedance electrode, the second lowest impedance electrode, and the third lowest impedance electrode are assigned to a left arm (LA) electrode, right arm (RA) electrode, and right leg drive (RLD) electrode based on a geometry of arrangement.

In some embodiments, the third lowest impedance electrode is assigned to an RLD electrode, and the lowest impedance electrode and the second lowest impedance electrode are assigned to an LA electrode and an RA electrode, where the LA electrode and the RA electrode are assigned based on a pattern matching of a signal with a standard ECG.

In some embodiments, the lowest impedance electrode, the second lowest impedance electrode, and the third lowest impedance electrode are assigned to an LA electrode, an RA electrode, and an RLD electode based on pattern matching a structure of a corresponding maternal ECG generated by a plurality of assignments comprising all combinations of assignment between the lowest impedance electrode, the second lowest impedance electrode, the third lowest impedance electrode, and the LA electrode, the RA electrode, and the RLD electode.

In some embodiments, pattern matching the structure includes selecting an assignment from the plurality of assignments based on the corresponding maternal ECG matching a predetermined pattern.

A method for generating an abdominal maternal electrocardiogram includes selecting a plurality of combinations comprising all three-electrode combinations of a right arm lead, left arm lead, and right leg drive lead for a plurality of electrodes of an abdominal electrode device, measuring a corresponding plurality of signal to noise ratios for the plurality of combinations, comparing the corresponding plurality of signal to noise ratios to identify a combination having a comparatively highest signal to noise ratio, and generating an abdominal maternal electrocardiogram using signals from the combination.

In some embodiments, the method includes determining that a noise level of a predetermined frequency exceeds a predetermined noise threshold, and in response to the determination, measuring a second corresponding plurality of signal to noise ratios for the plurality of combinations, comparing the second corresponding plurality of signal to noise ratios to identify a second combination having the comparatively highest signal to noise ratio, and generating the abdominal maternal electrodcardiogram by using the second combination.

In some embodiments, the method includes determining that a noise level of a predetermined frequency occurs over a period of time exceeding a predetermined time threshold, and in response to the determination, measuring a second corresponding plurality of signal to noise ratios for the plurality of combinations, comparing the second corresponding plurality of signal to noise ratios to identify a second combination having the comparatively highest signal to noise ratio, and generating the abdominal maternal electrodcardiogram by using the second combination.

In some embodiments, the method includes determining that a signal loss from the lowest impedance electrode and the second lowest impedance electrode occurs beyond a predetermined time threshold, and in response to the determination, measuring a second corresponding plurality of signal to noise ratios for the plurality of combinations, comparing the second corresponding plurality of signal to noise ratios to identify a second combination having the comparatively highest signal to noise ratio, and generating the abdominal maternal electrodcardiogram by using the second combination.

A computer-readable medium includes instructions that are executable by a processor to determine a plurality of impedance values for a corresponding plurality of electrodes for an abdominal electrode device in contact with an abdomen, determine a lowest impedance value of the plurality of impedance values, a second lowest impedance value of the plurality of impedance values, and a third lowest impedance value of the plurality of impedance values by comparing the plurality of impedance values, and generate an abdominal maternal electrodcardiogram by using a lowest impedance electrode having the lowest impedance value, a second lowest impedance electrode having the second lowest impedance value, and a third lowest impedance electrode having the third lowest impedance value.

In some embodiments, the instructions are executable by the processor to determine that a noise level at a predetermined frequency band exceeds a predetermined noise threshold, and in response to the determination, determine a second plurality of impedance values for the corresponding plurality of electrodes for the abdominal electrode device in contact with the abdomen, determine the lowest impedance value of the second plurality of impedance values, the second lowest impedance value of the second plurality of impedance values, and the third lowest impedance value of the second plurality of impedance values by comparing the second plurality of impedance values, and generate the abdominal maternal electrodcardiogram by using the lowest impedance electrode, the second lowest impedance electrode, and the third lowest impedance electrode.

In some embodiments, the instructions are executable by the processor to determine that a noise level at a predetermined frequency occurs over a period of time exceeding a predetermined time threshold, and in response to the determination, determine a second plurality of impedance values for the corresponding plurality of electrodes for the abdominal electrode device in contact with the abdomen, determine the lowest impedance value of the second plurality of impedance values, the second lowest impedance value of the second plurality of impedance values, and the third lowest impedance value of the second plurality of impedance values by comparing the second plurality of impedance values, and generate the abdominal maternal electrodcardiogram by using the lowest impedance electrode, the second lowest impedance electrode, and the third lowest impedance electrode.

In some embodiments, the instructions are executable by the processor to determine that a signal loss occurs beyond a predetermined time threshold, and in response to the determination, determine a second plurality of impedance values for the corresponding plurality of electrodes for the abdominal electrode device in contact with the abdomen, determine the lowest impedance value of the second plurality of impedance values, the second lowest impedance value of the second plurality of impedance values, and the third lowest impedance value of the second plurality of impedance values by comparing the second plurality of impedance values, and generate the abdominal maternal electrodcardiogram by using the lowest impedance electrode, the second lowest impedance electrode, and the third lowest impedance electrode.

In some embodiments, the lowest impedance electrode, the second lowest impedance electrode, and the third lowest impedance electrode are assigned to a left arm (LA) electrode, right arm (RA) electrode, and right leg drive (RLD) electrode based on a geometry of arrangement.

In some embodiments, the third lowest impedance electrode is assigned to an RLD electrode, and the lowest impedance electrode and the second lowest impedance electrode are assigned to an LA electrode and an RA electrode, where the LA electrode and the RA electrode are assigned based on a pattern matching of a signal with a standard ECG.

In some embodiments, the lowest impedance electrode, the second lowest impedance electrode, and the third lowest impedance electrode are assigned to an LA electrode, an RA electrode, and an RLD electode based on pattern matching a structure of a corresponding maternal ECG generated by a plurality of assignments comprising all combinations of assignment between the lowest impedance electrode, the second lowest impedance electrode, the third lowest impedance electrode, and the LA electrode, the RA electrode, and the RLD electode.

In some embodiments, pattern matching the structure comprises selecting an assignment from the plurality of assignments based on the corresponding maternal ECG matching a predetermined pattern.

Various other features, objects, and advantages of the invention will be made apparent from the following description taken together with the drawings.

### BRIEF DESCRIPTION OF THE DRAWINGS

The drawings illustrate the best mode presently contemplated of carrying out the disclosure. In the drawings:
Fig. 1 is a perspective view of an example abdominal electrode device with adaptive electrode selection for abdominal maternal electrocardiogram, according to some embodiments of the present disclosure.
Fig. 2 is a perspective view of an example abdominal electrode device with adaptive electrode selection for abdominal maternal electrocardiogram, according to some embodiments of the present disclosure.
Fig. 3 is a system of adaptive electrode selection for abdominal maternal electrocardiogram, according to some embodiments of the present disclosure.
Fig. 4 is a flow chart of an example method of adaptive electrode selection for abdominal maternal electrocardiogram, according to some embodiments of the present disclosure.
Fig. 5 is a flow chart of an example method of adaptive electrode selection for abdominal maternal electrocardiogram, according to some embodiments of the present disclosure.
Fig. 6 is an exemplary adaptive electrode selector, according to some embodiments of the present disclosure.

### DETAILED DESCRIPTION

In the present description, certain terms have been used for brevity, clarity and understanding. No unnecessary limitations are to be inferred therefrom beyond the requirement of the prior art because such terms are used for descriptive purposes only and are intended to be broadly construed.

As used herein, unless otherwise limited or defined, discussion of particular directions is provided by example only, with regard to particular embodiments or relevant illustrations. For example, discussion of "top," "bottom," "front," "rear," "left," "right," "horizontal," "vertical," and "longitudinal" features and/or relative motion, e.g., movement "up" and "down," is generally intended as a description only of the orientation of such features relative to a reference frame of a particular example or illustration. Correspondingly, for example, a "top" feature may sometimes be disposed below a "bottom" feature (and so on), in some arrangements or embodiments. Additionally or alternatively, embodiments may be arranged in a different orientation such that "top" and "bottom" features are arranged horizontally relative to each other, for example in a "left-to-right" orientation.

The use herein of the terms "including," "including," or "having," and variations thereof, is meant to encompass the elements listed thereafter and equivalents thereof, as well as additional elements. Embodiments recited as "including," "including," or "having" certain elements are also contemplated as "consisting essentially of" and "consisting of" those certain elements.

As stated previously, a probe for conducting an electrocardiogram test typically may include a number of electrodes that interface with the skin of the patient and acquire electric biopotentials from the heart. Being able to identify the electrical activity of the maternal heart may be useful for determining an accurate fetal heart rate. The fetal heart rate may be determined using a Doppler ultrasound device. However, when the maternal and fetal heartbeats coincide, readings from the Doppler ultrasound device may indicate the maternal heart rate instead of the fetal heart rate. Accordingly, the ECG may make it possible to identify when this coincidence occurs, thereby making it possible to distinguish the fetal heart rate from the maternal heart rate. In order to reduce the maternal patient's discomfort, and the number of probes and/or devices connected to her, the ECG electrodes may be incorporated into a tocodynamometer device, which is useful for measuring uterine contractions. This hybrid tocodynamometer device may include a wet ECG electrode, meaning the ECG electrode may be placed against the patient's skin with a conductive gel or sticker that improves the conductive contact between the electrode and the skin of the maternal abdomen or a dry electrode. Because the electrodes are incorporated into the tocodynamometer, and the tocodynamometer is placed on the maternal abdomen, the ECG test is referred to as an abdominal ECG.

The present inventors have identified problems with the available systems that can be improved upon. It has been observed by the inventors that, conductive gels and stickers may cause patient discomfort due to the potential for skin irritation from the the wet materials. Further, in a probe that integrates a dry electrode-based single-lead abdominal ECG into a tocodynamometer, there is the potential for failure to perform an accurate abdominal ECG. More specifically, to perform an accurate abdominal ECG, it is useful to maintain contact between the ECG electrode and the skin of the maternal abdomen. However, without a conductive gel or sticker to help maintain conductive contact between the ECG electrodes and the skin, the curvature of the abdomen may cause a reduction in the amount of contact between the ECG electrodes and the skin of the maternal abdomen. As such, the quality of the ECG signal may degrade, or the ECG electrodes may not conduct an ECG signal. Further, the ECG signal may degrade due to maternal movement, fetal movement, or other causes of signal artifacts. Thus, it may be useful to reposition the probe to improve the ECG signal. However, repositioning the probe may complicate the workflow of the healthcare provider, thus drawing their attention away from other priorities in the maternal patient's care. Further, it may be uncomfortable and/or otherwise stressful for the maternal patient to have the probe repeatedly repositioned.

Accordingly, in some embodiments of the present disclosure, the hybrid tocodynamometer may include multiple electrodes. Further, to improve the accuracy of the abdominal ECG over current techniques, such embodiments may use the ECG signals from the electrodes having the relatively highest skin contact among the multiple ECG electrodes that are incorporated into the hybrid tocodynamometer.

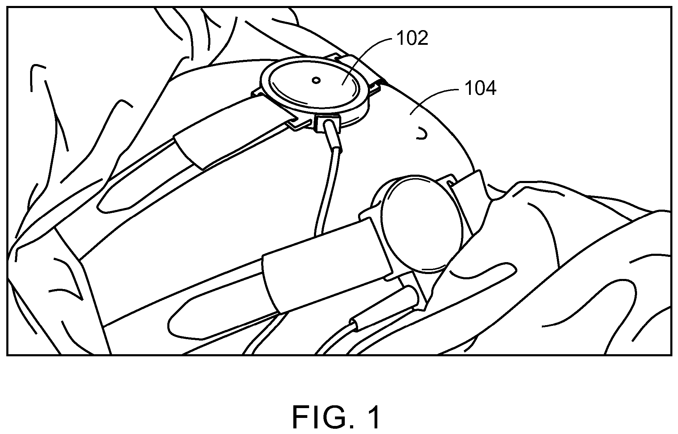

Fig. 1 is a perspective view of an example abdominal electrode device 102 with adaptive electrode selection for abdominal maternal electrocardiogram, according to some embodiments of the present disclosure. The example abdominal electrode device 102 may be a may be a hybrid tocodynamometer device that measures uterine activity and cardiac activity of a pregnant patient. More specifically, the example abdominal electrode device 102 may include multiple electrodes (not shown) that conduct potentials from the skin of the patient. Further, the example abdominal electrode device 102 may be configured to measure these potentials, and use these measurements to generate a maternal abdominal maternal electrocardiogram. In this example, the abdominal electrode device 102 is placed on the mother's abdomen 104. Since the abdomen 104 is not flat, the electrodes (not shown) may partially or fully lose contact with the skin, affecting signal quality. Accordingly, in some embodiments of the present disclosure, the contact impedance of each electrode is measured by scanning all the electrodes. Additionally, such embodiments may assign the electrodes with the three lowest impedance measures, respectively, to the left arm (LA), right arm (RA) and right leg drive (RLD) electrodes for the purpose of generating the abdominal maternal electrocardiogram. Alternatively, such embodiments may assign the electrodes with the three lowest impedance measures, respectively, to the right arm (RA), left arm (LA) and right leg drive (RLD) electrodes for the purpose of generating the abdominal maternal electrocardiogram. In other words, such embodiments may use the signals from the assigned RA, LA, and RLD electrodes to generate the abdominal maternal electrocardiogram as if the assigned electrodes were placed on the right arm, left arm, and right leg of the patient. Other alternatives to electrode assignment include, assigning the electrodes with the three lowest impedances as LA, RA and RLD based on geometry, where the relative positions of the electrodes determine which is LA, RA and RL; and, based on pattern matching the ECG signal itself. With pattern matching, the ECG is acquired with all possible assignments of LA, RA and RLD to the three electrodes with the lowest impedance. Accordingly, there are 6 possible combinations in which they can be assigned. Using pattern matching of the acquired ECG signals with a standard (reference) ECG signal, the combination that correctly defines LA, RA and RLD can be identified. Thus, the assignment of the LA, RA, and RLD electrodes depnds on the phase of the signal, amplitude of the signal, and the width of the QRS (e.g., Q, R, and S waves of the signal).

Further, according to some embodiments of the present disclosure, the electrode assignments may be based on signal to noise ratio. More specifically, such embodiments may calculate the signal-to-noise ratio for every three-electrode combination of the electrodes on the example abdominal electrode device 102. Accordingly, such embodiments may generate the abdominal maternal electrocardiogram using the three-electrode combination with the comparatively highest signal-to-noise ratio.

Additionally, according to some embodiments of the present disclosure, the electrode selection and assignment may be performed under two conditions. The first condition may be an initial state of the example abdominal electrode device, meaning a predetermined time after the example abdominal electrode device 102 is powered on and/or placed on the abdomen 104 of the patient.

Further, such embodiments may perform selection and assignment when relatively low-frequency noise (e.g., below 5 Hz) exceeds a predetermined threshold. Alternatively, or additionally, such embodiments may perform assignment and selection when signal noise (e.g., low-frequency noise) endures beyond a predetermined threshold of time. Such conditions may indicate potential changes in electrode-skin contact, meaning the current assignment of electrodes may provide a lack of, or diminished, signals, thus degrading the quality of the corresponding abdominal maternal electrocardiogram. Thus, by reselecting and assigning the electrodes under these conditions, such embodiments may generate the abdominal maternal electrocardiogram using the electrodes with the highest relative skin contact. By using multiple electrodes and using the signals from the electrodes having the highest relative skin contact, such embodiments may improve the reliability and accuracy of maternal heart rate monitoring over current techniques. In these ways, some embodiments of the present disclosure may mitigate the issue of electrode contact loss due to the abdomen's curvature, thus improving the quality of the abdominal ECG readings, and reducing the likelihood of repositioning the example abdominal electrode device. Additionally, the use of dry electrodes may improve the ease of use for healthcare providers, and improve the comfort for patients, as dry electrodes are not used with conductive gels that may cause skin irritation, and lengthen the preparation time. As a result, healthcare providers can achieve more precise and consistent patient monitoring, lesser preparation time, leading to better-informed clinical decisions and improved patient outcomes. For patients, this may mean a higher level of care, comfort, and confidence in the accuracy of their health data, which may improve the patients' overall well-being.

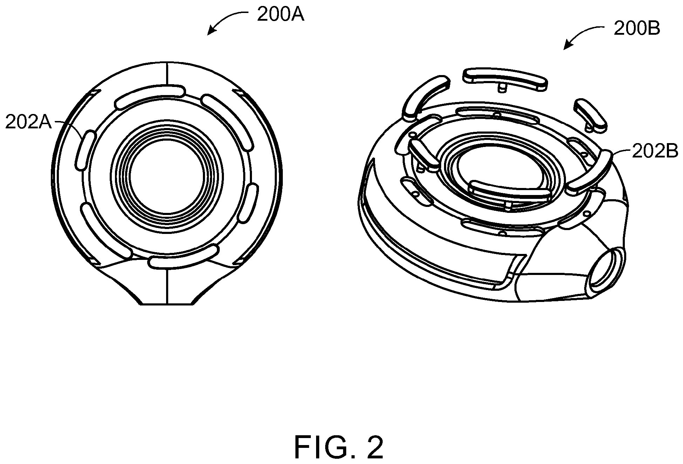

Fig. 2 is a perspective view of example abdominal electrode devices 200A, 200B with adaptive electrode selection for abdominal maternal electrocardiogram, according to some embodiments of the present disclosure. The example abdominal electrode devices 200A, 200B are similar to the example abdominal electrode device 102, described with respect to Fig. 1. Further, the example abdominal electrode device 200A includes example electrodes 202A. Additionally, the example abdominal electrode device 200B represents an exploded view of the example abdominal electrode device 200B with example electrodes 202B. As stated previously, some embodiments of the present disclosure may measure the contact impedance of each electrode by scanning all the electrodes 202A, 202B. Additionally, such embodiments may assign the electrodes with the three lowest impedance measures, respectively, to the left arm (LA), right arm (RA) and right leg drive (RLD) electrodes for the purpose of generating the abdominal maternal electrocardiogram. Alternatively, such embodiments may assign the electrodes with the three lowest impedance measures, respectively, to the right arm (RA), left arm (LA) and right leg drive (RLD) electrodes for the purpose of generating the abdominal maternal electrocardiogram.

Alternatively, some embodiments of the present disclosure may select the electrodes for generating the abdominal maternal electrocardiogram based on signal to noise ratio. As stated previously, such embodiments may calculate the signal-to-noise ratio for every three-electrode combination of the electrodes 202A, 202B on the example abdominal electrode device 200A, 200B. Further, such embodiments may generate the abdominal maternal electrocardiogram using the three-electrode combination with the comparatively highest signal-to-noise ratio.

Additionally, according to some embodiments of the present disclosure, the electrode selection and assignment may be performed during an initial state of the example abdominal electrode device 200A, 2000B, e.g., a predetermined time after the example abdominal electrode device 200A, 200B is powered on and/or placed on the abdomen of the patient. Further, such embodiments may perform selection and assignment when relatively low-frequency noise exceeds a predetermined threshold, and/or when signal noise endures beyond a predetermined threshold of time. As stated previously, such conditions may indicate potential changes in electrode-skin contact, meaning the current assignment of electrodes may provide degraded (or no) signals. In these ways, such embodiments may provide an abdominal maternal electrocardiogram that represents an improvement over electrocardiograms provided by current systems using dry electrodes.

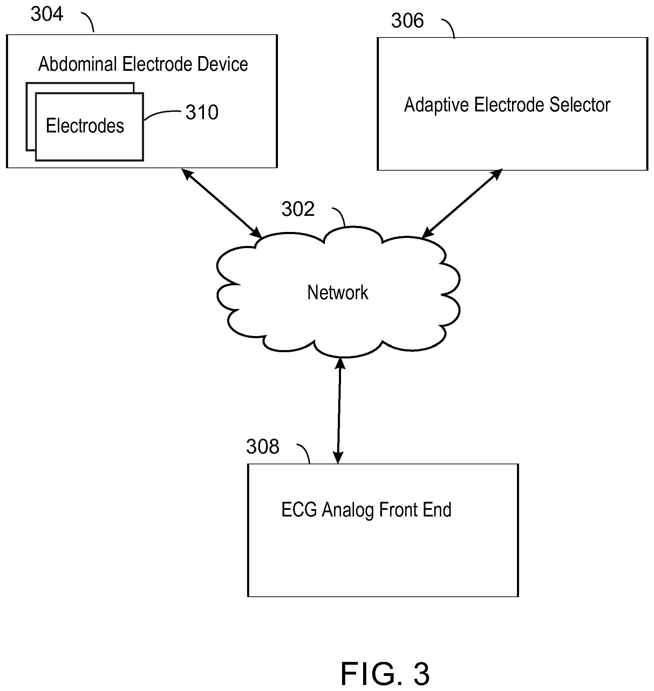

Fig. 3 is a system 300 of adaptive electrode selection for abdominal maternal electrocardiogram, according to some embodiments of the present disclosure. The system 300 includes a network 302, example abdominal electrode device 304, example adaptive electrode selector 306, and electrocardiogram (ECG) analog front end 308. The network 302 maybe one or more computer communication networks that facilitate communication between the example abdominal electrode device 304 and example adaptive electrode selector 306. According to some embodiments of the present disclosure, the network 302 may be a wired or wireless network, and may include a local area network, a wide area network, and the like. In some examples, the network 302 may be the Internet. The example abdominal electrode device 304 may be similar to the example abdominal electrode devices 102, 200A, 200B, described with respect to Figs. 1 and 2. Accordingly, the example abdominal electrode device 304 may include multiple electrodes 310 arranged on a tocodynamometer enclosure. The electrodes 310 may be similar to the electrodes 202A, 202B, described with respect to Fig. 2. As stated previously, the electrodes 310 may measure ECG signals from the abdominal area. According to some embodiments of the present disclosure, each electrode of a single-lead ECG (i.e., LA, RA, and RLD) could be assigned to any electrode 310. Further, the example abdominal electrode device 304 may include multiple electrodes 310. Additionally, the example adaptive electrode selector 306 may be a set of computer-readable instructions that are execuable by a computer processor to adaptively select electrodes of the example abdominal electrode device 304 for generating an abdominal maternal electrocardiogram for a pregnant patient. Accordingly, the example adaptive electrode selector 306 may select the electrodes whose measurements are used to generate the maternal abdominal maternal electrocardiogram based on the impedance values of the individual electrodes 310 and/or the signal-to-noise ratios of all combinations of the electrodes 310.

The system 300 is merely one example of an adaptive electrode selection system, according to some embodiments of the present disclosure. Alternatively, an adaptive electrode selection system may not include the network 302. Rather, the example abdominal electrode device 304 may include the example adaptive electrode selector 306, ECG analog front end 308, and electrodes 310. The ECG analog front end 308 may receive the analog ECG signals from the hybrid tocodynamometer, process the analog ECG signals, and convert the analog ECG signals to digital signals.

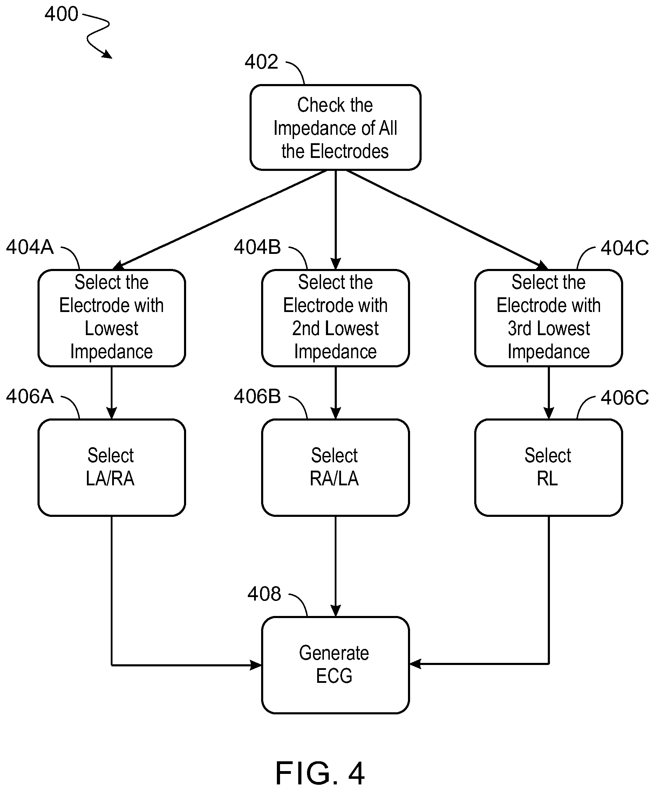

Fig. 4 is a flow chart of an example method 400 of adaptive electrode selection for abdominal maternal electrocardiogram, according to some embodiments of the present disclosure. In this example, an adaptive electrode selector such as, the adaptive electrode selector 306 and the ECG analog front end 308, may perform the method 400.

At operation 402, the adaptive electrode selector 306 may determine the impedance values of all the electrodes (e.g., electrodes 310) of an abdominal electrode device (e.g., the example abdominal electrode device 306).

The ratio of voltage drop to current injected by an electrode is equal to two times the contact impedance of the electrode 310 that injects the current. Accordingly, the contact impedance of an electrode 310 is equal to the voltage drop divided by two, and multiplied by the current injected. This calculation is represented in EQUATION 1 below: $Contact impedance = \frac{Voltage Drop}{2 ∗ Current Injected}$ EQUATION 1

The voltage drop may be the difference between the voltage of the injected current, and the measure of the injected current across two electrodes (e.g., two different electrodes from the electrode whose impedance is being measured). Accordingly, for each of the electrodes 310, the adaptive electrode selector 306 may cause the electrode 310 to inject a predetermined amount of current into the skin of the patient. Additionally, the adaptive electrode selector 306 may measure the voltage of the conducted current across two other electrodes 310. Further, the adaptive electrode selector 306 may determine the voltage drop by subtracting the measured voltage from the voltage of the injected current. Accordingly, the adaptive electrode selector 306 may determine the contact impedance for the electrode according to EQUATION 1.

At operation 404A, the adaptive electrode selector 306 may identify the electrode 310 having the comparatively lowest contact impedance. Additionally, at operation 406A, the adaptive electrode selector 306 may assign the identified electrode to the left arm or right arm electrode.

At operation 404B, the adaptive electrode selector 306 may identify the electrode 310 having the comparatively second lowest contact impedance. Additionally, at operation 406B, the adaptive electrode selector 306 may assign the identified electrode to the other of the left arm or right arm electrode assigned at operation 406A. In other words, if, at operation 406A, the adaptive electrode selector 306 assigns the identified electrode to the right arm, at operation 406B, the adaptive electrode selector 306 assigns the identified electrode to the left arm.

At operation 404C, the adaptive electrode selector 306 may identify the electrode 310 having the comparatively third lowest contact impedance. Additionally, at operation 406C, the adaptive electrode selector 306 may assign the identified electrode to the right leg drive electrode.

With respect to operations 404A, 404B, 404C, the electrode assignment may vary as described previously. More specifically, the adaptive electrode selector 306 may assign the electrodes with the three lowest impedances as LA, RA and RLD based on geometry, where the relative positions of the electrodes determine which is LA, RA and RL; and, based on pattern matching the ECG signal itself. With pattern matching, the ECG is acquired with all possible assignments of LA, RA and RLD to the three electrodes with the lowest impedance. Accordingly, there are 6 possible combinations in which they can be assigned. As stated previously, using pattern matching of the acquired ECG signals with a standard (reference) ECG signal, the combination that correctly defines LA, RA and RLD can be identified.

At operation 408, the ECG analog front end 308 may generate the ECG based on the signals from the electrodes 310 and their assignments. The operation 408 may repeat with the same electrode selection until low-frequency noise exceeds a predetermined threshold, or signal loss occurs for a predetermined duration. Such scenarios may indicate movement and/or changes in electrode-skin contact. Accordingly, in such scenarios, the adaptive electrode selector may repeat the method 400.

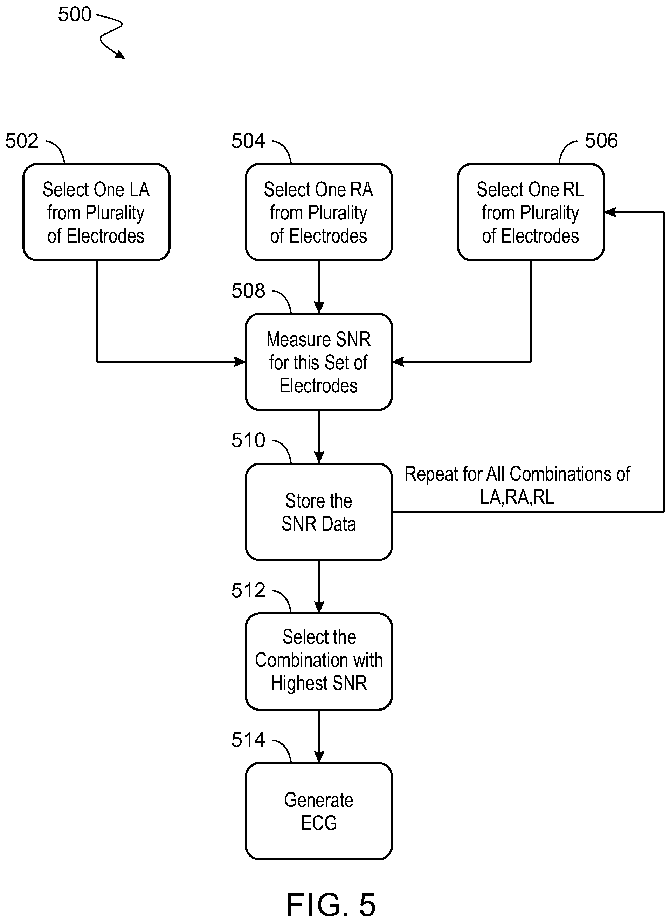

Fig. 5 is a flow chart of an example method 500 of adaptive electrode selection for abdominal maternal electrocardiogram, according to some embodiments of the present disclosure. The method 500 may be performed by the adaptive electrode selector 306 and the ECG analog front end 308. According to some embodiments of the present disclosure, the adaptive electrode selector 306 may repeat operations 502 through 510 for each left arm, right arm, right leg drive assignment combination of electrodes 310.

At operation 502, the adaptive electrode selector 306 may select one electrode 310 to represent the left arm electrode. At operation 504, the adaptive electrode selector 306 may select another electrode 310 to represent the right arm electrode. At operation 506, the adaptive electrode selector 306 may select another electrode 310 to represent the right leg drive electrode.

At operation 508, the adaptive electrode selector may measure the signal-to-noise ratio for the selected set of electrodes 310. More specifically, within a window of a predetermined duration of an ECG signal, the adaptive electrode selector 306 may calculate the frequency response of the ECG signal. Further, the adaptive electrode selector 306 may find the power of the actual signal by considering the frequency band of a typical ECG signal (e.g., from 5 Hz to 40 Hz), as indicated in EQUATION 2, where P represents the power, f represents the frequency, and X represents the signal in the frequency domain. $P = {\int }_{- ∞}^{∞} P \left(f\right) df = {\int }_{- ∞}^{∞} {\left|X\left(f\right)\right|}^{2} df$ EQUATION 2

Accordingly, the adaptive electrode selector 306 may find the power of the noise signal for remaining frequency band by excluding the typical ECG frequency band during integration. Further, the adaptive electrode selector 306 may find the ratio of powers for signal and noise, as indicated in EQUATION 3: ${SNR}_{dB} = 10 {log}_{10} \left(\frac{{P}_{signal}}{{P}_{noise}}\right)$ EQUATION 3

At operation 510, the adaptive electrode selector may store the measured signal-to-noise ratio for the selected set of electrodes. Further, the control of the method 500 may flow back to operation 502 to repeat for another set of electrodes. In this way, operations 502 through 510 may repeat for all combinations of electrode assignment to left arm, right arm, and right leg drive electrodes.

At operation 512, the adaptive electrode selector 306 may select the right arm, left arm, right leg drive electrode assignment with the comparatively highest signal-to-noise ratio measurement of all the electrode combinations.

At operation 514, the ECG generator 508 may generate the ECG based on the selected electrode combination. The operation 514 may repeat with the same electrode selection until low-frequency noise exceeds a predetermined threshold, or signal loss occurs for a predetermined duration. Such scenarios may indicate movement and/or changes in electrode-skin contact. Accordingly, in such scenarios, the adaptive electrode selector 306 may repeat the method 500.

In these ways, some embodiments of the present disclosure may enable the generation of a more reliable and accurate ECG than possible with current methods, regardless of which electrodes maintain effective contact with the skin, even when the surface of the skin is not flat. Accordingly, such embodiments may reduce repositioning of the abdominal electrode device 304 to maintain effective contact of the electrodes 310. In these ways, such embodiments may provide improved signal acquisition. By maintaining contact, the abdominal electrode device 304 may provide relatively continuous and reliable signal acquisition, which is useful for accurate ECG readings. This reduces the likelihood of signal loss or noise due to poor electrode to skin contact. Additionally, such embodiments may provide improve user comfort and convenience in the use of electrodes for ECG. User comfort may be improved by reducing adjustments in the positioning of the electrodes This also may be beneficial in long-term monitoring scenarios, where repositioning can be inconvenient and uncomfortable for the patient.

Further, monitoring capabilities may be improved because generating ECGs with the electrodes 310 having the relatively greater skin contact, the abdominal electrode device can provide more accurate and reliable monitoring than possible with current techniques, which is useful for diagnosing and managing cardiac conditions. This can lead to better patient outcomes and more efficient use of clinical resources.

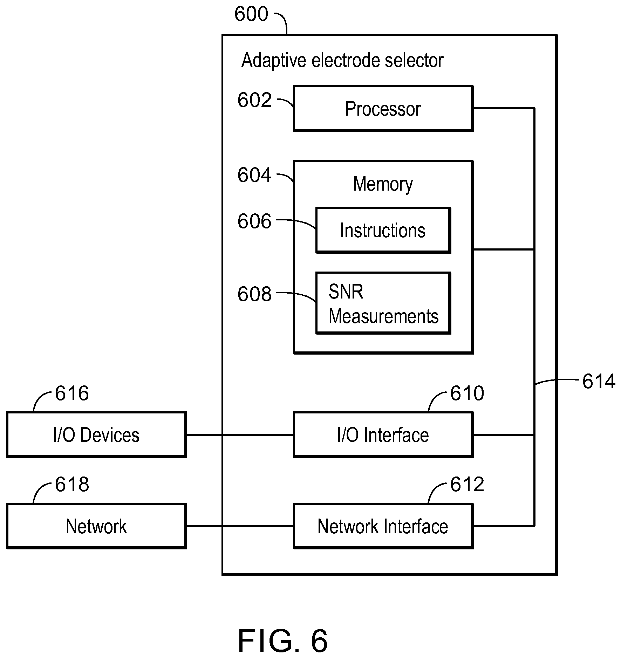

Fig. 6 is an exemplary adaptive electrode selector 600, according to some embodiments of the present disclosure. The example adaptive electrode selector 600 may perform adaptive electrode selection as described with respect to Figs. 1-5. In this example, the adaptive electrode selector 600 includes a processor 602, memory 604, input-output (I/O) interface 610, and network interface 612, which may be connected by an interconnect 614. The processor 602 may be a computer processing circuit (e.g., a central processing unit (CPU)) that retrieves and executes programming instructions 606 stored in the memory 604 to perform the functionality described herein. The interconnect 614 may move data, such as programming instructions, between the processor 602, memory 604, I/O interface 610, and network interface 612. The interconnect 614 may include one or more buses.

The memory 604 may be a computer memory or storage device, including volatile memory, such as a random access memory (RAM) device (e.g., static RAM, dynamic RAM, and the like), non-volatile memory, such as a hard disk drive, solid state device (SSD), removable memory cards, optical storage, flash memory devices, and the like. In some examples, the memory 604 may include volatile and non-volatile memory devices. Further, the memory 604 may store instructions 606, which may cause the processor to perform adaptive electrode selection as described with respect to Figs. 1-5.

Additionally, the adaptive electrode selector 600 may be in electronic communication with I/O devices 616 through the I/O interface 610, and with a network 618 through the network interface 612. The I/O devices 616 may capture inputs and provide outputs as described herein. The network 618 may be an electronic communication network, such as a local area network, wide area network, and the like, for processing communications between the adaptive electrode selector 600 and the abdominal electrode device, e.g., abdominal electrode devices 102, 200A, 200B, 304, described herein. In some examples, the network 618 may be wired, wireless (e.g., wi-fi, Bluetooth, or cellular), or some other computer communication network.

In some embodiments, the adaptive electrode selector 600 may be a server computer or similar device without a user interface but which receives requests from other computer systems having one or more user interfaces. Further, in some embodiments, the adaptive electrode selector 600 may be a portable computer, laptop, tablet computer, pocket computer, telephone, smart phone, or the like.

An example system for adaptive electrode selection for abdominal maternal electrocardiogram, including a processor and a memory. The memory includes instructions executable by the processor to determine impedance values for electrodes of an abdominal electrode device in contact with an abdomen, determine a lowest impedance value of the impedance values, a second lowest impedance value of the impedance values, and a third lowest impedance value of the impedance values by comparing the impedance values, generate an abdominal maternal electrodcardiogram by using a lowest impedance electrode having the lowest impedance value, a second lowest impedance electrode having the second lowest impedance value, and a third lowest impedance electrode having the third lowest impedance value.

In some examples, the instructions are executable by the processor to determine that a noise level at a predetermined frequency band exceeds a predetermined noise threshold, and in response to the determination, determine a second plurality of impedance values for the corresponding plurality of electrodes for the abdominal electrode device in contact with the abdomen, determine the lowest impedance value of the second plurality of impedance values, the second lowest impedance value of the second plurality of impedance values, and the third lowest impedance value of the second plurality of impedance values by comparing the second plurality of impedance values, and generate the abdominal maternal electrodcardiogram by using the lowest impedance electrode, the second lowest impedance electrode, and the third lowest impedance electrode.

In some examples, the instructions are executable by the processor to determine that a noise level at a predetermined frequency occurs over a period of time exceeding a predetermined time threshold, in response to the determination, determine a second plurality of impedance values for the corresponding plurality of electrodes for the abdominal electrode device in contact with the abdomen, determine the lowest impedance value of the second plurality of impedance values, the second lowest impedance value of the second plurality of impedance values, and the third lowest impedance value of the second plurality of impedance values by comparing the second plurality of impedance values, and generate the abdominal maternal electrodcardiogram by using the lowest impedance electrode, the second lowest impedance electrode, and the third lowest impedance electrode.

In some examples, the instructions are executable by the processor to determine that a signal loss occurs beyond a predetermined time threshold, in response to the determination, determine a second plurality of impedance values for the corresponding plurality of electrodes for the abdominal electrode device in contact with the abdomen, determine the lowest impedance value of the second plurality of impedance values, the second lowest impedance value of the second plurality of impedance values, and the third lowest impedance value of the second plurality of impedance values by comparing the second plurality of impedance values, and generate the abdominal maternal electrodcardiogram by using the lowest impedance electrode, the second lowest impedance electrode, and the third lowest impedance electrode.

In some examples, the lowest impedance electrode, the second lowest impedance electrode, and the third lowest impedance electrode are assigned to a left arm (LA) electrode, right arm (RA) electrode, and right leg drive (RLD) electrode based on a geometry of arrangement.

In some examples, the third lowest impedance electrode is assigned to an RLD electrode, and the lowest impedance electrode and the second lowest impedance electrode are assigned to an LA electrode and an RA electrode, where the LA electrode and the RA electrode are assigned based on a pattern matching of a signal with a standard ECG.

In some examples, the lowest impedance electrode, the second lowest impedance electrode, and the third lowest impedance electrode are assigned to an LA electrode, an RA electrode, and an RLD electode based on pattern matching a structure of a corresponding maternal ECG generated by a plurality of assignments comprising all combinations of assignment between the lowest impedance electrode, the second lowest impedance electrode, the third lowest impedance electrode, and the LA electrode, the RA electrode, and the RLD electode.

In some examples, pattern matching the structure includes selecting an assignment from the plurality of assignments based on the corresponding maternal ECG matching a predetermined pattern.

An example method for generating an abdominal maternal electrocardiogram includes selecting a plurality of combinations comprising all three-electrode combinations of a right arm lead, left arm lead, and right leg drive lead for a plurality of electrodes of an abdominal electrode device, measuring a corresponding plurality of signal to noise ratios for the plurality of combinations, comparing the corresponding plurality of signal to noise ratios to identify a combination having a comparatively highest signal to noise ratio, and generating an abdominal maternal electrocardiogram using signals from the combination.

In some examples, the method includes determining that a noise level of a predetermined frequency exceeds a predetermined noise threshold, and in response to the determination, measuring a second corresponding plurality of signal to noise ratios for the plurality of combinations, comparing the second corresponding plurality of signal to noise ratios to identify a second combination having the comparatively highest signal to noise ratio, and generating the abdominal maternal electrodcardiogram by using the second combination.

In some examples, the method includes determining that a noise level of a predetermined frequency occurs over a period of time exceeding a predetermined time threshold, and in response to the determination, measuring a second corresponding plurality of signal to noise ratios for the plurality of combinations, comparing the second corresponding plurality of signal to noise ratios to identify a second combination having the comparatively highest signal to noise ratio, and generating the abdominal maternal electrodcardiogram by using the second combination.

In some examples, the method includes determining that a signal loss from the lowest impedance electrode and the second lowest impedance electrode occurs beyond a predetermined time threshold, and in response to the determination, measuring a second corresponding plurality of signal to noise ratios for the plurality of combinations, comparing the second corresponding plurality of signal to noise ratios to identify a second combination having the comparatively highest signal to noise ratio, and generating the abdominal maternal electrodcardiogram by using the second combination.

An example computer-readable medium includes instructions that are executable by a processor to determine a plurality of impedance values for a corresponding plurality of electrodes for an abdominal electrode device in contact with an abdomen, determine a lowest impedance value of the plurality of impedance values, a second lowest impedance value of the plurality of impedance values, and a third lowest impedance value of the plurality of impedance values by comparing the plurality of impedance values, and generate an abdominal maternal electrodcardiogram by using a lowest impedance electrode having the lowest impedance value, a second lowest impedance electrode having the second lowest impedance value, and a third lowest impedance electrode having the third lowest impedance value.

In some examples, the instructions are executable by the processor to determine that a noise level at a predetermined frequency band exceeds a predetermined noise threshold, and in response to the determination, determine a second plurality of impedance values for the corresponding plurality of electrodes for the abdominal electrode device in contact with the abdomen, determine the lowest impedance value of the second plurality of impedance values, the second lowest impedance value of the second plurality of impedance values, and the third lowest impedance value of the second plurality of impedance values by comparing the second plurality of impedance values, and generate the abdominal maternal electrodcardiogram by using the lowest impedance electrode, the second lowest impedance electrode, and the third lowest impedance electrode.

In some examples, the instructions are executable by the processor to determine that a noise level at a predetermined frequency occurs over a period of time exceeding a predetermined time threshold, and in response to the determination, determine a second plurality of impedance values for the corresponding plurality of electrodes for the abdominal electrode device in contact with the abdomen, determine the lowest impedance value of the second plurality of impedance values, the second lowest impedance value of the second plurality of impedance values, and the third lowest impedance value of the second plurality of impedance values by comparing the second plurality of impedance values, and generate the abdominal maternal electrodcardiogram by using the lowest impedance electrode, the second lowest impedance electrode, and the third lowest impedance electrode.

In some examples, the instructions are executable by the processor to determine that a signal loss occurs beyond a predetermined time threshold, and in response to the determination, determine a second plurality of impedance values for the corresponding plurality of electrodes for the abdominal electrode device in contact with the abdomen, determine the lowest impedance value of the second plurality of impedance values, the second lowest impedance value of the second plurality of impedance values, and the third lowest impedance value of the second plurality of impedance values by comparing the second plurality of impedance values, and generate the abdominal maternal electrodcardiogram by using the lowest impedance electrode, the second lowest impedance electrode, and the third lowest impedance electrode.

In some examples, the lowest impedance electrode, the second lowest impedance electrode, and the third lowest impedance electrode are assigned to a left arm (LA) electrode, right arm (RA) electrode, and right leg drive (RLD) electrode based on a geometry of arrangement.

In some examples, the third lowest impedance electrode is assigned to an RLD electrode, and the lowest impedance electrode and the second lowest impedance electrode are assigned to an LA electrode and an RA electrode, where the LA electrode and the RA electrode are assigned based on a pattern matching of a signal with a standard ECG.

In some examples, the lowest impedance electrode, the second lowest impedance electrode, and the third lowest impedance electrode are assigned to an LA electrode, an RA electrode, and an RLD electode based on pattern matching a structure of a corresponding maternal ECG generated by a plurality of assignments comprising all combinations of assignment between the lowest impedance electrode, the second lowest impedance electrode, the third lowest impedance electrode, and the LA electrode, the RA electrode, and the RLD electode.

In some examples, pattern matching the structure comprises selecting an assignment from the plurality of assignments based on the corresponding maternal ECG matching a predetermined pattern.

As used herein, the term, mechanism, can encompass hardware, software, firmware, or any suitable combination thereof. In some embodiments, any suitable computer readable media can be used for storing instructions for performing functions and/or processes described herein. For example, in some embodiments, computer readable media can be transitory or non-transitory. For example, non-transitory computer readable media can include media such as magnetic media (such as tape, hard disks, floppy disks, ands the like), optical media (such as compact discs, digital video discs, Blu-ray discs, and the like), semiconductor media (such as RAM, Flash memory, electrically programmable read only memory (EPROM), electrically erasable programmable read only memory (EEPROM), and the like), any suitable media that is not fleeting or devoid of any semblance of permanence during transmission, and/or any suitable tangible media. As another example, transitory computer readable media can include signals on networks, in wires, conductors, optical fibers, circuits, or any suitable media that is fleeting and devoid of any semblance of permanence during transmission, and/or any suitable intangible media.

This written description uses examples to disclose the invention, including the best mode, and also to enable any person skilled in the art to make and use the invention. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims.

## Claims

1. A system for adaptive electrode selection for abdominal maternal electrocardiogram, comprising:
a processor; and
a memory including instructions executable by the processor to:
determine a plurality of impedance values for a corresponding plurality of electrodes for an abdominal electrode device in contact with an abdomen;
determine a lowest impedance value of the plurality of impedance values, a second lowest impedance value of the plurality of impedance values, and a third lowest impedance value of the plurality of impedance values by comparing the plurality of impedance values; and
generate an abdominal maternal electrodcardiogram by using a lowest impedance electrode having the lowest impedance value, a second lowest impedance electrode having the second lowest impedance value, and a third lowest impedance electrode having the third lowest impedance value.

2. The system of claim 1, the instructions being executable by the processor to:
determine that a noise level at a predetermined frequency band exceeds a predetermined noise threshold; and
in response to the determination:
determine a second plurality of impedance values for the corresponding plurality of electrodes for the abdominal electrode device in contact with the abdomen;
determine the lowest impedance value of the second plurality of impedance values, the second lowest impedance value of the second plurality of impedance values, and the third lowest impedance value of the second plurality of impedance values by comparing the second plurality of impedance values; and
generate the abdominal maternal electrodcardiogram by using the lowest impedance electrode, the second lowest impedance electrode, and the third lowest impedance electrode.

3. The system of claim 1, the instructions being executable by the processor to:
determine that a noise level at a predetermined frequency occurs over a period of time exceeding a predetermined time threshold; and
in response to the determination:
determine a second plurality of impedance values for the corresponding plurality of electrodes for the abdominal electrode device in contact with the abdomen;
determine the lowest impedance value of the second plurality of impedance values, the second lowest impedance value of the second plurality of impedance values, and the third lowest impedance value of the second plurality of impedance values by comparing the second plurality of impedance values; and
generate the abdominal maternal electrodcardiogram by using the lowest impedance electrode, the second lowest impedance electrode, and the third lowest impedance electrode.

4. The system of claim 1, the instructions being executable by the processor to:
determine that a signal loss occurs beyond a predetermined time threshold; and
in response to the determination:
determine a second plurality of impedance values for the corresponding plurality of electrodes for the abdominal electrode device in contact with the abdomen;
determine the lowest impedance value of the second plurality of impedance values, the second lowest impedance value of the second plurality of impedance values, and the third lowest impedance value of the second plurality of impedance values by comparing the second plurality of impedance values; and
generate the abdominal maternal electrodcardiogram by using the lowest impedance electrode, the second lowest impedance electrode, and the third lowest impedance electrode.

5. The system of claim 1, wherein the lowest impedance electrode, the second lowest impedance electrode, and the third lowest impedance electrode are assigned to a left arm (LA) electrode, right arm (RA) electrode, and right leg drive (RLD) electrode based on a geometry of arrangement.

6. The system of claim 1, wherein:
the third lowest impedance electrode is assigned to an RLD electrode; and
the lowest impedance electrode and the second lowest impedance electrode are assigned to an LA electrode and an RA electrode, where the LA electrode and the RA electrode are assigned based on a pattern matching of a signal with a standard ECG.

7. The system of claim 1, wherein the lowest impedance electrode, the second lowest impedance electrode, and the third lowest impedance electrode are assigned to an LA electrode, an RA electrode, and an RLD electode based on pattern matching a structure of a corresponding maternal ECG generated by a plurality of assignments comprising all combinations of assignment between the lowest impedance electrode, the second lowest impedance electrode, the third lowest impedance electrode, and the LA electrode, the RA electrode, and the RLD electode.

8. The system of claim 7, wherein pattern matching the structure comprises selecting an assignment from the plurality of assignments based on the corresponding maternal ECG matching a predetermined pattern.

9. A method for generating an abdominal maternal electrocardiogram, comprising:
selecting a plurality of combinations comprising all three-electrode combinations of a right arm lead, left arm lead, and right leg drive lead for a plurality of electrodes of an abdominal electrode device;
measuring a corresponding plurality of signal to noise ratios for the plurality of combinations;
comparing the corresponding plurality of signal to noise ratios to identify a combination having a comparatively highest signal to noise ratio; and
generating an abdominal maternal electrocardiogram using signals from the combination.

10. The method of claim 9, comprising:
determining that a noise level of a predetermined frequency exceeds a predetermined noise threshold; and
in response to the determination:
measuring a second corresponding plurality of signal to noise ratios for the plurality of combinations;
comparing the second corresponding plurality of signal to noise ratios to identify a second combination having the comparatively highest signal to noise ratio; and
generating the abdominal maternal electrodcardiogram by using the second combination.

11. The method of claim 9, comprising:
determining that a noise level of a predetermined frequency occurs over a period of time exceeding a predetermined time threshold; and
in response to the determination:
measuring a second corresponding plurality of signal to noise ratios for the plurality of combinations;
comparing the second corresponding plurality of signal to noise ratios to identify a
second combination having the comparatively highest signal to noise ratio; and generating the abdominal maternal electrodcardiogram by using the second combination.

12. The method of claim 9, comprising:
determining that a signal loss from the lowest impedance electrode and the second lowest impedance electrode occurs beyond a predetermined time threshold; and
in response to the determination:
measuring a second corresponding plurality of signal to noise ratios for the plurality of combinations;
comparing the second corresponding plurality of signal to noise ratios to identify a second combination having the comparatively highest signal to noise ratio; and
generating the abdominal maternal electrodcardiogram by using the second combination.
